# EUROPEAN PATENT APPLICATION

(11) **EP 4 290 237 A1**
(43) Date of publication of application: **13.12.2023**
(21) Application number: 22382565.4
(22) Date of filing: 10.06.2022
(51) Int. Cl.: G01N 33/564, G01N 33/50

(54) **METHOD FOR DETERMINING RESPOSE TO METHROTREXATE (MTX) IN A HUMAN SUBJECT DIAGNOSED WITH RHEUMATOID ARTHRITIS**

(71) Applicant: Biohope Scientific Solutions for Human Health SL, 28760 Tres Cantos (Madrid) (ES)
(72) Inventor: YEBRA POLO, Tatiana, 28760 Tres Cantos (ES); ORTEGA CARRIÓN, Alvaro, 28760 Tres Cantos (ES); ZANOTTI, Carlo, 28760 Tres Cantos (ES); PORTERO SÁNCHEZ, Isabel María, 28760 Tres Cantos (ES)
(74) Representative: Hoffmann Eitle

(57) **Abstract**

The present invention relates to the medical field, specifically to the diagnostic/predictive field using biomarkers of response to methotrexate (MTX) in a human subject diagnosed with rheumatoid arthritis.

## Description

### Technical field

The present invention relates to the medical field, specifically to the diagnostic/predictive field using biomarkers of response to methotrexate (MTX) in a human subject diagnosed with rheumatoid arthritis.

### Background of the invention

Methotrexate (MTX) is an anti-metabolite that possesses anti-proliferative and immunosuppressive activity by competitively inhibiting the enzyme dihydrofolate reductase, which catalyzes a key step in folic acid metabolism by regulating the amount of intracellular folate available for protein and nucleic acid synthesis. In the 1950s it began to be used for the treatment of cancer, particularly acute leukemias, and for the last 25 years it has been used, at lower doses than those used in oncology, for the treatment of various autoimmune diseases.

From the regulatory point of view, MTX has indications approved both in Europe and in other regions as an oncological treatment (mainly leukemias) and for diseases of the immune system. As regards the latter, the indications currently approved are (according to the AEMPS (Spanish Agency of Medicines and Medical Devices) technical data sheet):
- active rheumatoid arthritis in adult patients,
- polyarthritic forms of severe active juvenile idiopathic arthritis, when the response to non-steroidal anti-inflammatory drugs (NSAIDs) has not been adequate,
- severe recalcitrant and disabling psoriasis that does not respond adequately to other treatments such as phototherapy, PUVA and retinoids,
- severe psoriatic arthritis in adult patients,
- mild to moderate Crohn's disease, alone or in combination with corticosteroids, in refractory or thiopurine-intolerant adult patients.

Currently, MTX is one of the first-line treatments for rheumatoid arthritis (RA).

Remission is the ideal therapeutic goal in rheumatoid arthritis. To define remission, criteria are needed to differentiate the presence or absence of activity and to be sufficiently reliable to support therapeutic decisions. There are currently many different criteria, with DAS28 (Disease Activity Score, the DAS28 index has established itself as a key variable for assessing the activity of rheumatoid arthritis and is the main parameter used to establish therapeutic decisions in this disease, including the start and change of biologic treatments) being one of the criteria validated by the EULAR/ACR (European League Against Rheumatism/American College of Rheumatology), and the most widely accepted, indicating disease activity in 28 joints.
- Interpretation of the DAS28:

| | |
|---|---|
| DAS28 <2.6 | Remission |
| DAS28 >=2.6 and <= 3.2: | Low disease activity |
| DAS28 >3.2 and <=5.1: | Moderate disease activity |
| DAS28 >5.1 | High disease activity |

Another criterion accepted by EULAR is the SDAI (Simple Disease Activity Index):

| | |
|---|---|
| SDAI <=3.3 | Remission |
| SDAI >=3.3 and <= 11: | Low disease activity |
| SDAI 11 and <=26 | Moderate disease activity |
| SDAI >26 | High disease activity |

The SDAI considers the same joints as the DAS28 and therefore the ability to classify patients is very similar to that of the DAS. The SDAI includes the physician's assessment of disease activity on a scale of 1-10.

These criteria have a very clear objective, to help in the diagnosis and prognostic stratification of RA at early stages, so that treatment with disease-modifying drugs (DMARDs), including methotrexate, can be established. However, even though MTX is a first-line drug, widely used in the treatment of RA, one third of patients with RA do not respond to this treatment. In this sense, a tool is needed to detect those patients who, prior to starting treatment with MTX, will not respond adequately to methotrexate after 6 months of continuous treatment and, therefore, it is necessary to identify those patients who are at high risk of developing a persistent or erosive disease. This is because adequate treatment in the early stages of the disease is essential to prevent a joint destructive process that starts very early, in the first weeks or months, and thus prevent a sustained inflammatory activity over time responsible for all the consequences of the disease. It should be considered that, to date, there is no biomarker available to anticipate the clinical response to MTX in a patient diagnosed with RA before starting treatment.

Thus, the present invention provides assays developed with the intention of measuring the response to methotrexate of a patient with RA prior to starting treatment with said drug, providing different and complementary results.

### Brief description of the figures

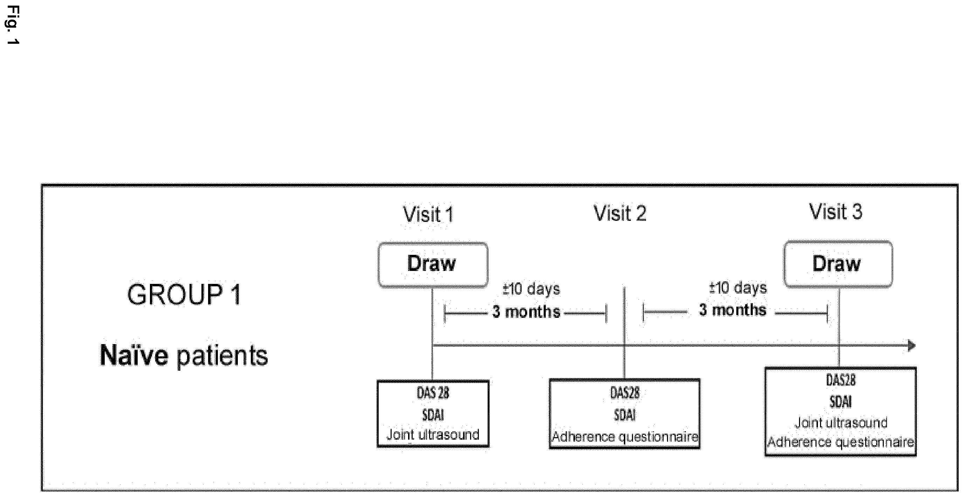

Figure 1. Diagram of scheduled visits per patient according to the methodology indicated in the examples.

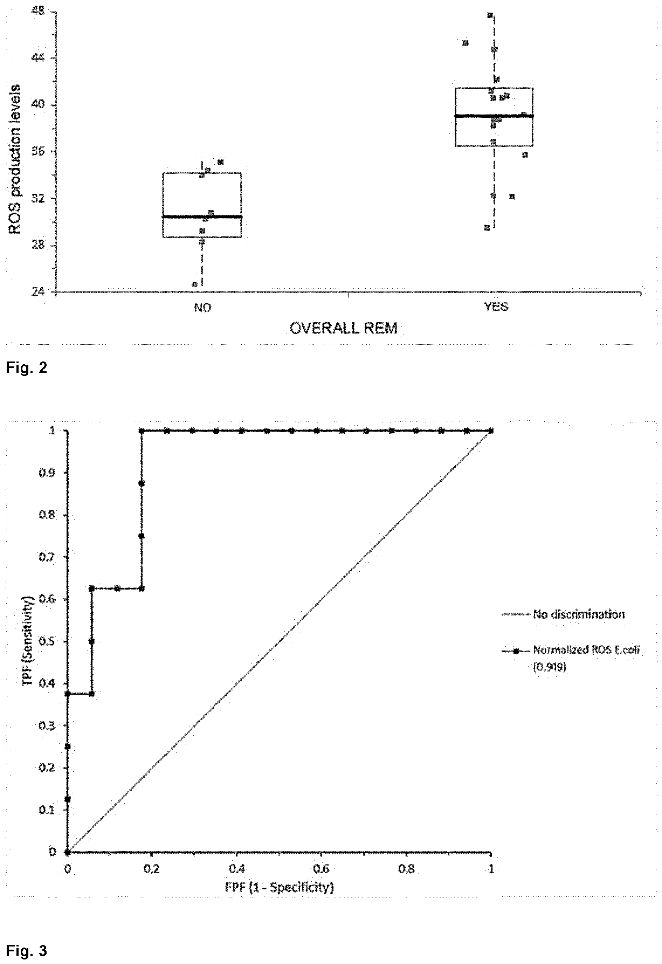

**Figure 2****.** ROS production (fluorescence units) of PBMCs from, NO: naïve RA patients who do not present clinical remission at 6 months of treatment with methotrexate (n= 8), YES: naïve RA patients who do present clinical remission at 6 months of treatment with methotrexate (n= 17) (total n= 25).
**Figure 3****.** ROC curve plot of the ROS test in patients with new-onset rheumatoid arthritis who did and did not present clinical remission in response to methotrexate. Each of the points represents a possible cut-off point of the diagnostic test and reports its respective sensitivity (y-axis) and specificity (x-axis). Both axes include values between 0 and 1.

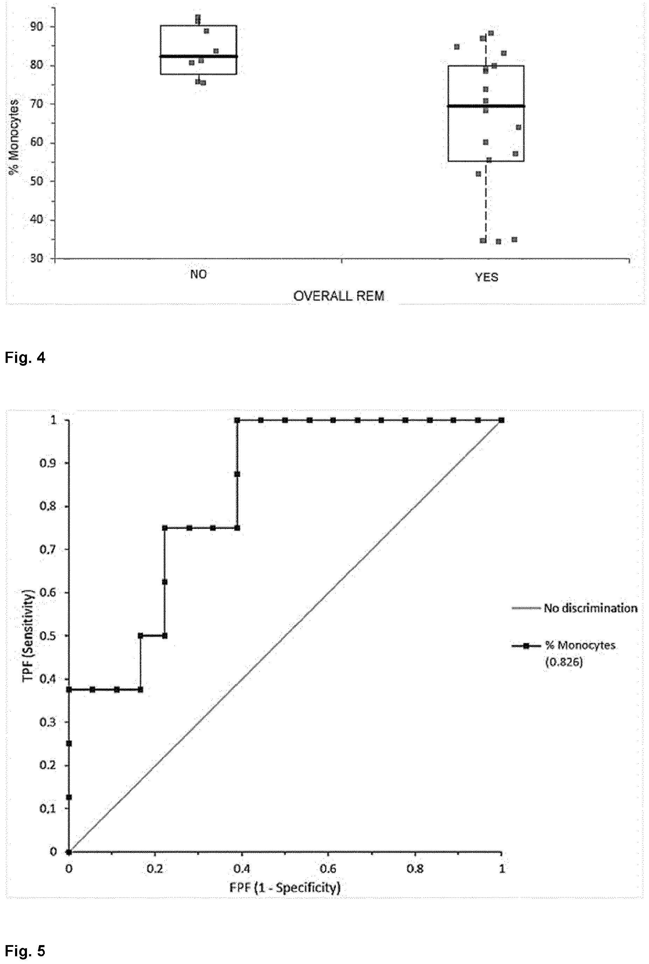

**Figure 4****.** Percentage of monocytes from, NO: naive RA patients who do not present clinical remission at 6 months of treatment with methotrexate (n= 8), YES: naive RA patients who do present clinical remission at 6 months of treatment with methotrexate (n= 18) (total n= 26).
**Figure 5****.** ROC curve plot of the monocyte test in patients with new-onset rheumatoid arthritis who did and did not present clinical remission in response to methotrexate. Each of the points represents a possible cut-off point of the diagnostic test and reports its respective sensitivity (y-axis) and specificity (x-axis). Both axes include values between 0 and 1.

### Description of the invention

### Definitions

In the context of the present invention, activated "T lymphocytes and/or monocytes" are understood as cells of the immune system which through various signals can differentiate into effector lymphocytes and/or activated macrophages and/or effector cells derived therefrom. As used herein, "methotrexate" or "MTX" is understood as a disease-modifying drug, used alone or with other drugs for the treatment of rheumatoid arthritis, with multiple mechanisms of action through which it has the ability to reduce autoimmune joint inflammation and inflammation in any other tissue that is subject to immune attack in the context of the disease.

As used here, "reactive oxygen species" or "ROS" is understood as unstable molecules or free radicals containing oxygen that react readily with other molecules in cells. These species are generated from physiological cellular metabolism.

As used here, rheumatoid arthritis or "RA" is understood as a heterogeneous, complex, autoimmune disease with joint and possibly systemic involvement in numerous organs and tissues, with highly variable phenotypic, serologic and inflammatory marker expression. In its most classic form (but not the only one, nor necessarily the most frequent), it is characterized by an episodic, chronic, erosive, deforming, symmetrical polyarthritis that leads to long-term joint disability if not controlled.

As used here, "disease-modifying drugs" or "DMARDs" are understood as a heterogeneous group of drugs widely used in the treatment of rheumatoid arthritis that have the potential to positively influence the prognosis and long-term progression of the disease. It is a very heterogeneous group of drugs according to their chemical composition and mechanism of action.

### Description

MTX has effects on T-cell proliferation both *in vitro* and *in vivo.* At low doses, this effect is not so much at the cytotoxic level, but rather the cytostatic level. In clinical practice, the efficacy of low doses of methotrexate usually takes some time, as the accumulation of intracellular polyglutamated methotrexate is a slow process. The polyglutamated form of methotrexate is believed to be responsible for its activity as a DMARD.

One hypothesis, among the various mechanisms of action attributed to methotrexate in its use for autoimmune diseases is redox alteration, an important mechanism in the immunosuppressive action of the drug. MTX at low doses has been found to induce apoptosis of transformed T cells through the generation of reactive oxygen species (ROS). ROS are involved not only in acting against pathogens or cell death, but also modulate different cellular functions, such as suppression of cytokine production and cell proliferation. Contrary to the conventional view that elevated ROS levels are involved in inflammation, it has been shown that reducing the ability to produce ROS promotes the activation of arthritogenic T cells leading to severe arthritis in murine models (4). MTX-induced ROS generation may be mediated by depletion of tetrahydrobiopterin. MTX is a potent inhibitor of DHFR. DHFR catalyzes the reduction of dihydrobiopterin to tetrahydrobiopterin which is a necessary cofactor for NO synthesis by nitric oxide synthases (NOS). Depletion of tetrahydrobiopterin uncouples NOS, leading to loss of NO synthesis and increased ROS production. Both modulation of IL-6 synthesis and ROS production contribute to the therapeutic effects of MTX for RA (5). However, these findings have been demonstrated in murine models and immortalized lymphocyte lines, but have never been consolidated in human lymphocytes isolated from peripheral blood, even less in patients with diagnosed RA. The study that comes closest to the stated hypothesis has been demonstrated in samples from patients with psoriasis, where MTX induced apoptosis through oxidative stress by reducing NO and increasing caspase-3 levels. It is this induced apoptosis that explains the beneficial effect of treatment with MTX in these patients (6).

Based on the foregoing and as detailed in the examples, the present invention shows, for the first time, a test referred to as the "ROS TEST". Activated immune system cells typically generate reactive oxygen species, to a greater or lesser extent depending on the cell type and its function. This invention tests a casual and not necessarily predicted observation that increased redox activity is paradoxically shown in cultured PBMCs from MTX-responsive RA patient cells. Therefore, the hypothesis is that treatment of PBMCs with the drug methotrexate causes an increase in reactive oxygen species (ROS) production due to increased apoptosis in T lymphocytes in responder patients. This increase could serve as a biomarker of response to treatment in patients diagnosed with RA before starting said treatment.

As shown in the examples, the above hypothesis was demonstrated by the authors of the present invention; in fact, Figure 2 shows the distribution of all patients tested in the examples, including patients with RA responders and non-responders to MTX. The normal distribution of the results was tested by the D'Agostino Pearson test. Comparisons and differences between groups were analyzed by ANOVA analysis. Specifically, the ANOVA statistical analysis showed a statistically significant difference between those naive RA patients who did not present clinical remission at 6 months of treatment with methotrexate and those naïve RA patients who did present clinical remission at 6 months of treatment with methotrexate. That is, ROS production levels, measured after exposure of PBMCs to MTX, were significantly increased in those naïve RA patients who presented clinical remission at 6 months of treatment with methotrexate versus those who did not present clinical remission. On the other hand, Figure 3 shows a ROC curve plot of the ROS test in patients with recent-onset rheumatoid arthritis who did and did not present clinical remission in response to methotrexate. Each of the points represents a possible cut-off point of the diagnostic test and reports its respective sensitivity (y-axis) and specificity (x-axis). Both axes include values between 0 and 1. The AUC in Figure 3 presents a clearly discriminatory value higher than 0.90.

Therefore, from this the following can be concluded:
- There is a clear association between ROS production in naïve patients and response to methotrexate.
- Patients able to metabolize MTX at low doses maintain the drug by-products intracellularly for a time, during which time ROS detectable by the assay are generated.

Therefore, a first aspect of the invention relates to the use of ROS (reactive oxygen species) production levels measured, preferably by fluorimetry or by flow cytometry, *in vitro* from a biological sample that has been or is in contact with methotrexate (MTX), wherein the biological sample comprises peripheral blood mononuclear cells (PBMCs), or a population of T lymphocytes and/or monocytes, preferably a substantially pure population of activated T lymphocytes and/or monocytes, and isolated from a human subject diagnosed with rheumatoid arthritis and characterized in that the subject has not, prior to obtaining the sample, been treated with disease-modifying drugs (DMARDs), such as MTX, as a biomarker of response of said subject to methotrexate. In the present invention, "response" of the subject to MTX is understood as clinical remission at 3 months, preferably at 6 months from treatment with methotrexate. "Clinical remission" is preferably understood as the absence of inflammatory activity or sufficiently mild inflammatory activity so as not to compromise the patient's functional activity, preferably on a long-term basis. Preferably, such clinical remission can be measured using clinical signs of joint inflammation or serological parameters. More preferably, this definition can be translated into a preferably significant improvement in various response indices, including any one of the following: DAS28, SDAI, CDAI, "Boolean" definitions, combinations thereof, or others. In the experimentation presented, the DAS28 criterion has been selected because it is a widely validated criterion and one of the most widely used in clinical practice worldwide. Therefore, even more preferably, "clinical remission" is understood as the subject or patient presenting at 3 months, preferably at 6 months, from the start of treatment with methotrexate, a DAS28 score below the 2.6 score and/or a reduction of at least 1.2 points, with respect to the start of treatment with methotrexate, in the DAS28 score.

In a particular embodiment of the first aspect of the invention, the human subject from which the sample is obtained is characterized in that the subject has not been treated with MTX, and preferably in that the subject presents a DAS 28 ≥ 2.6.

In another particular embodiment of the first aspect of the invention or any of its particular embodiments, the biological sample of PBMCs or T lymphocytes and/or monocytes is activated prior to or simultaneously upon contact of the biological sample with MTX with an activating compound, preferably by mitogens, such as, but not limited to: PHA (phytohemagglutinin), bacterial antigens such as LPS (lipopolysaccharide), T-lymphocyte stimulating antibodies (such as anti-CD3 and/or anti-CD28), concanavalin A or phorbol esters (PMA) plus ionomycin.

In another particular embodiment of the first aspect of the invention or any of its particular embodiments, the biological sample is contacted with a concentration range of MTX in distilled water, saline or saline buffer of between 50 and 150 µg/µL, preferably of between 75 and 125 µg/µL, more preferably of between 90 and 110 µg/µL, still more preferably of about 100 µg/µL.

In another particular embodiment of the first aspect of the invention or any of its particular embodiments, the biological sample of PBMCs is obtained from a peripheral blood draw of the subject, and isolation of the peripheral blood mononuclear cells (PBMCs) is preferably performed by density gradient. However, alternatively, isolation of PBMCs can also be performed by:
- Density gradient;
- Isopycnic sedimentation; or
- Counter-flow centrifugation (CFC);
or by any other process that ensures the separation of mononuclear cells from plasma, granulocytes and the erythrocyte part as:
- By an osmotic shock technique to discard erythrocytes with positive immunoselection of PBMCs; or
- Negative immunoselection of neutrophils and NK and B lymphocytes.

As indicated above, the use of the first aspect of the invention can also be performed with a biological sample comprising leukocyte subpopulations obtained by immunoselection isolation of T lymphocytes and/or monocytes.

In another particular embodiment of the first aspect of the invention or any of its particular embodiments, ROS (reactive oxygen species) production levels measured *in vitro* from a biological sample of activated PBMCs or T lymphocytes and/or monocytes exposed to or contacted with MTX are measured by fluorimetry, using developer reagents such as fluorogenic substrates or ROS detection probes that are localized in the cytoplasm or in the cell nucleus and mitochondria and detectable in equipment capable of reading multiwell plates, or by flow cytometry, using hydroethidine (HE), dihydrorhodamine (DHR123), or other probes capable of detecting intracellular reactive oxygen species. Some specific probes useful for performing detection by fluorimetry are RED ROS or Amplex RED, or by flow cytometry, using hydroethidine (HE), dihydrorhodamine (DHR123), or Cell ROX Green Orange. Preferably, the developer reagent is RED ROS which is prepared in solution maintaining the ratio of 2.5 µL of ROS probe per milliliter of saline buffer.

A second aspect of the invention relates to a method for determining the response to methotrexate (MTX) of a subject diagnosed with RA (rheumatoid arthritis) from a sample of PBMCs isolated from said subject, or a population of T lymphocytes and/or monocytes, preferably a substantially pure population of activated T lymphocytes and/or monocytes, wherein the subject is characterized in that the subject has not, prior to obtaining the sample, been treated with disease-modifying drugs (DMARDs), such as MTX, and wherein the method comprises:
a. Determining or measuring the ROS (reactive oxygen species) production levels of the biological sample, in contact with a sufficient amount of methotrexate (MTX), from peripheral blood mononuclear cells (PBMCs) or activated T lymphocytes and/or monocytes isolated from the human subject diagnosed with rheumatoid arthritis;
b. Comparing the result of the determination of step a) with a reference value indicative of ROS production in a subject or population of subjects not responding to MTX, where values, preferably significantly, higher than such reference value are indicative of response to treatment with MTX and that the subject is therefore a responder to such treatment. Otherwise, it will be understood that the subject is a non-responder to MTX.

It is noted that, alternatively to comparing with a reference value indicative of ROS production in a subject or population of subjects not responding to MTX, one could compare with a population preferably of PBMCs, or in any other sample that is of the same nature as the analyte (subject or patient sample), previously induced for ROS production by ROS production inducers such as, for example, pyocyanins, N-formyl-Met-Lau-Phe (fMLP), tert-butyl hydroperoxide (TBHP), etc., and without MTX stimulus.

Again, it is noted that, in the present invention, "response" of the subject to MTX is understood as clinical remission at 3 months, preferably at 6 months from the start of the subject's treatment with methotrexate. "Clinical remission" is preferably understood as the absence of inflammatory activity or sufficiently mild or declining inflammatory activity so as not to compromise functional activity, preferably on a long-term basis. Preferably, such clinical remission can be measured using clinical signs of joint inflammation or serological parameters. More preferably, this definition can be translated into a preferably significant improvement in various response indices, including any one of the following: DAS28, SDAI, CDAI, "Boolean" definitions, combinations thereof, or others. In the experimentation presented, the DAS28 criterion has been selected because it is a widely validated criterion and one of the most widely used in clinical practice worldwide. Therefore, even more preferably, "clinical remission" is understood as the subject or patient presenting at 3 months, preferably at 6 months, from the start of treatment with methotrexate, a DAS28 score below the 2.6 score and/or a reduction of at least 1.2 points, with respect to the start of treatment with methotrexate, in the DAS28 score.

In a particular embodiment of the second aspect of the invention, the human subject from which the sample is obtained is characterized in that the subject has not been treated with MTX, and preferably in that the subject presents a DAS 28 ≥ 2.6.

It is noted that the biological sample of the second aspect of the invention comprises PBMCs or T lymphocytes and/or monocytes, preferably a substantially pure population of T lymphocytes and/or monocytes, i.e., a population comprising at least 80%, 85%, 90%, 95%, 96%, 97%, 98% or 99% T lymphocytes and/or monocytes. It is noted that for obtaining the biological sample comprising PBMCs or T lymphocytes and/or monocytes, a volume of whole blood from the subject of between 2 mL and 10 mL is preferably drawn. Such peripheral blood draw will usually be deposited in blood tubes containing anticoagulants (sodium heparin, lithium heparin and EDTA).

Once whole blood has been obtained from the subject, preferably isolation of peripheral blood mononuclear cells (PBMCs) by density gradient will be performed from the blood drawn from the subject. However, alternatively, isolation of PBMCs can be performed by:
- Density gradient.
- Isopycnic sedimentation; or
- Counter-flow centrifugation (CFC);
or by any other process that ensures the separation of mononuclear cells from plasma, granulocytes and the erythrocyte part as:
- By an osmotic shock technique to discard erythrocytes with positive immunoselection of PBMCs; or
- Negative immunoselection of neutrophils and NK and B lymphocytes.

As indicated above, the method of the second aspect of the invention can also be performed with a biological sample comprising leukocyte subpopulations obtained by immunoselection isolation of T lymphocytes and/or monocytes.

In a preferred embodiment of the second aspect of the invention, preferably the PBMCs or T lymphocytes and/or monocytes once obtained and prior to their exposure or contact with MTX and the corresponding ROS measurement, are frozen (preferably 7 to 12 million per 1 mL vial) and maintained, preferably in liquid nitrogen, for a time period of at least one or two weeks. For this purpose, preferably the cell suspension in culture medium, containing the PBMCs or T lymphocytes and/or monocytes isolated from the subject, is mixed with a cryopreservation liquid (such as for example a mixture of fetal or human bovine serum, cell culture medium and DMSO) and maintained, preferably in liquid nitrogen, for at least one week and up to one year. In a preferred embodiment freezing can be carried out by automatic systems with continuous cell freezing ramp.

In another preferred embodiment of the second aspect of the invention, and assuming that PBMCs or T lymphocytes and/or monocytes have been frozen prior to their exposure or contact with MTX and the corresponding ROS measurement, these cells are thawed after a time period of at least one or two weeks from the time such cells were frozen. It is noted that thawing, if carried out, can be done by a rapid process, centrifuging and re-suspending the pellet in a cell culture medium with pH between 7 and 8, preferably supplemented with 1% AB human serum. Alternatively, this can be carried out in a water bath at 37°C for 2-3 minutes. Once the cells are thawed, preferably, and as mentioned above, all or part of the subject cell volume contained in the thawed cryovial is transferred to a container, preferably a sterile cell culture medium centrifuge tube, and centrifuged.

In a preferred embodiment the PBMCs or T lymphocytes and/or monocytes obtained from the subject, once thawed assuming the freezing step has been performed, are maintained in a supplemented medium or in 3D cell matrices: hydrogels and/or hydrophilic semi-solid matrices that allow cell maintenance and solubilization of the reagents used in the technique. Preferably, the supplemented medium is prepared by adding approximately 1% serum, preferably human serum type AB in order to maintain the cells once thawed. The reason for the above is because a recovery time of the culture is advisable after thawing because the cells need to equilibrate osmotically and at the cell membrane level for preferably a minimum time period of 12 hours and up to 24 hours. An instantaneous cell treatment could lead to nonspecific apoptosis. Human serum is suitable for physiological and natural conditions *in vitro* to be maintained similar to in vivo conditions and, therefore, the present invention proposes using the supplemented medium consisting of a medium for *in vitro* cell growth supplemented with human serum type AB at 1%, preferably, after centrifugation and once the supernatant has been discarded, to resuspend the pellet and add the supplemented medium. Once this step is completed, preferably cell counting and calculation of the total number of PBMCs or viable T lymphocytes and/or monocytes available are performed. For this purpose, preferably a cell count of viable mononuclear cells can be carried out, using a Neubauer chamber, an automatic hematological counter or any other method capable of discriminating viable cells from non-viable cells.

In another preferred embodiment of the second aspect of the invention, once the cell suspension comprising PBMCs or T lymphocytes and/or monocytes has been obtained, preferably in a medium suitable for this purpose such as a supplemented medium or in 3D cell matrices, preferably at a concentration of at least 4 million cells/mL, said cell suspension is dispensed onto a plate or support suitable for carrying out the test or method of the second aspect of the invention. For this purpose, preferably a multiwell plate (microtiter) is used, most preferably a transparent, flat-bottomed p96 plate (alternatively, other plates such as p48, p24, p12, or p6 or any other suitable plate can be used as a support for carrying out the method of the second aspect of the invention).

Preferably, the sample is homogenized and dispensed into a multiwell plate, preferably using the following volumes (starting from a concentration of at least 4 million cells/mL):
- p96 plate: 50 - 300 µL /well (preferably approximately 100 µL /well).
- p48 plate: 500-1500 µL /well
- p24 plate: 1500-3000 µL /well
- p12 plate: 4000-6500 µL /well
- p6 plate: 10-15 mL/well

Preferably the wells on the sides are covered with sterile distilled water to prevent evaporation of the liquid contained in the inner wells. It should be noted that as an alternative to the plate, any other suitable device or support such as a cell culture flask with a filter or sterile tubes or culture bags that allow gas exchange can be used.

In another preferred embodiment of the second aspect of the invention, once the cell suspension comprising the PBMCs or T lymphocytes and/or monocytes has been dispensed onto a suitable support for carrying out the method of the second aspect of the invention, such as a multiwell plate, said support is incubated, preferably in an incubator at approximately 37°C and 5% CO₂ atmosphere for preferably a time period of between 12 to 24 hours.

Next, and once the wells of the plate or any other alternative carrier comprise the cell suspension, the cell suspension comprised in one or more locations or well(s) of said carrier is contacted with MTX prior to, simultaneously or subsequent to activation of the cells in the cell suspension with a T lymphocyte and/or monocyte activator, preferably by mitogens, such as, but not limited to: PHA (phytohemagglutinin), bacterial antigens such as LPS (lipopolysaccharide), T-lymphocyte stimulating antibodies (such as anti-CD3 and/or anti-CD28), Concanavalin A or phorbol esters (PMA) plus ionomycin.

MTX preparation can preferably be performed by mixing methotrexate in distilled water, saline or saline buffer to a concentration of 100 µg/µL and about 5uL are added per well in p96 plate.

In another particular embodiment of the second aspect of the invention or any of its particular embodiments, the biological sample is contacted with a concentration range of MTX in distilled water, saline or saline buffer of between 50 and 150 µg/µL, preferably of between 75 and 125 µg/µL, more preferably of between 90 and 110 µg/µL, still more preferably of about 100 µg/µL.

If PHA is used as a cell activator, it is preferably prepared in solution by adding 400-600 µg of phytohemagglutinin to 1 mL of cell culture and approximately 1 µL of PHA solution is added to each well.

It is noted that preferably, the support where the assay or ROS test of the second aspect of the invention is carried out, such as in a multiwell plate, will contain one or more locations or wells that serve as controls of the assay such as one location or well where there is no exposure to the activating compound or MTX (containing only cell culture medium (white wells)) and another where only the cell suspension and the activating compound are exposed or contacted.

In a particular embodiment of the present invention, after contacting the MTX and/or activating compound, mitogen, with the T lymphocytes and/or monocytes, the support is incubated for 36 to 50 hours at approximately 37°C and 5% CO₂.

In a preferred embodiment of the present invention, the levels of reactive oxygen species (ROS (reactive oxygen species) production levels) necessary to implement the methodology of the second aspect are measured by fluorimetry, using developer reagents such as fluorogenic substrates or ROS detection probes that are localized in the cytoplasm or in the cell nucleus and mitochondria and detectable in equipment capable of reading multiwell plates, or by flow cytometry, using hydroethidine (HE), dihydrorhodamine (DHR123), or other probes capable of detecting intracellular reactive oxygen species. Preferably, the developer reagent is RED ROS which is prepared in solution maintaining the ratio of 2.5 µL of ROS probe per milliliter of saline buffer. Preferably, once the developer reagent has been added, the support or multiwell plate is incubated for 40-50 minutes and then the ROS levels are measured or read (for example by means of a fluorimeter adapted to multiwell plates).

In a preferred embodiment, to enhance the measurement of ROS levels preferably, subsequent to 40-50 minutes of incubation, a ROS enhancer such as a saline buffer solution with Escherichia coli at a final concentration of 400 to 1000 bacteria/ml could be added to the cell suspension. Other ROS stimuli derived from E. coli such as lipopolysaccharide could be used. In this scenario, ROS levels would be measured 10 to 20 minutes after exposure of the test sample to the ROS enhancer.

A third aspect of the invention relates to a kit for implementing the method for determining the response to methotrexate (MTX) of a subject diagnosed with RA according to the second aspect of the invention.

On the other hand, the present invention, in addition to the "ROS TEST" described above in the first and second aspects of the invention, refers to an additional assay or test called "MONOCYTE TEST". This test can be performed simultaneously or sequentially or concatenated to the "ROS TEST" as described in the first or second aspects of the invention.

Monocytes are key cells in the pathogenesis of diseases such as rheumatoid arthritis. Their activation and migration to the synovial membrane is a central phenomenon in the onset of inflammatory manifestations of the disease. They are cells of the innate immune system that have the capacity to bridge to adaptive immunity. These cells are probably one of the important targets for MTX. In this sense, the absolute number of monocytes in patients with rheumatoid arthritis who are non-responders should be increased (with respect to responders and healthy donors) at 6 months of treatment with MTX. However, the absolute number of monocytes may not be sensitive enough to detect interindividual differences with sufficient precision. Therefore, the authors of the present invention decided to investigate a model of *in vitro* exposure of RA patients' cells to MTX and the measurement of its direct effect as a pharmacodynamic biomarker of response. This test (MONOCYTE TEST), therefore, consists of a monocyte culture that is exposed to MTX (except for an active control). The overall cellular metabolic activity of the monocytes and their proliferation is subsequently measured using the resazurin reduction assay, which is a known and validated redox-sensitive fluorochrome that changes color according to the "metabolic intensity" and the number of live cells present. Activated and proliferating immune system cells exhibit a much more intense metabolic state, making it a good marker of overall response. Since MTX should inhibit cell activation and proliferation, comparison between the overall metabolism of active control cells vs. those exposed to MTX should provide a measure of the effect of MTX on monocyte activation and proliferation, which would serve as a biomarker of clinical response to MTX.

In fact, as shown in the examples of the present invention, Figure 4 shows the percentage of monocytes from, NO: naive RA patients who do not present clinical remission at 6 months of treatment with methotrexate (n= 8), SI: naive RA patients who do present clinical remission at 6 months of treatment with methotrexate (n= 18). The ANOVA analysis comparing these two groups revealed a clearly significant p-value that allowed to conclude a clear discriminatory capacity of this test to differentiate between naive RA patients who do not present clinical remission at 6 months of treatment with methotrexate and naive RA patients who do present clinical remission at 6 months of treatment with methotrexate. This data is corroborated by Figure 5 which shows an AUC of 0.826 (discriminatory value).

Therefore, a fourth aspect of the invention relates to the use of cellular metabolic activity levels obtained *in vitro* from an isolated biological sample of activated monocytes, which have been or are in contact with a sufficient amount of methotrexate (MTX), from a human subject diagnosed with rheumatoid arthritis and characterized in that the subject has not, prior to obtaining the sample, been treated with disease-modifying drugs (DMARDs), such as MTX, as a biomarker of response of said subject to methotrexate.

In the present invention, "response" of the subject to MTX is understood as clinical remission at 3 months, preferably at 6 months, from the start of treatment with methotrexate. "Clinical remission" is preferably understood as the absence of inflammatory activity or sufficiently mild inflammatory activity so as not to compromise functional activity, preferably on a long-term basis. Preferably, such clinical remission can be measured using clinical signs of joint inflammation or serological parameters. More preferably, this definition can be translated into a preferably significant improvement in various response indices, including any one of the following: DAS28, SDAI, CDAI, "Boolean" definitions, combinations thereof, or others. In the experimentation presented, the DAS28 criterion has been selected because it is a widely validated criterion and one of the most widely used in clinical practice worldwide. Therefore, even more preferably, "clinical remission" is understood as the subject or patient presenting at 3 months, preferably at 6 months, from the start of treatment with methotrexate, a DAS28 score below the 2.6 score and/or a reduction of at least 1.2 points, with respect to the start of treatment with methotrexate, in the DAS28 score.

In a particular embodiment of the fourth aspect of the invention, cellular metabolic activity is carried out with a fluorescence assay that measures cellular metabolic activity, such as with resazurin.

In another particular embodiment of the fourth aspect of the invention or any of its particular embodiments, the human subject from which the sample is obtained is characterized in that the subject has not been treated with MTX, and preferably in that the subject presents a DAS 28 ≥ 2.6.

In another particular embodiment of the fourth aspect of the invention or any of its particular embodiments, the biological sample comprises monocytes or a substantially pure population of monocytes, a substantially pure population of monocytes being understood as a population comprising at least 80%, 85%, 90%, 95%, 96%, 97%, 98% or 99% monocytes.

In another particular embodiment of the fourth aspect of the invention or any of its particular embodiments, the biological sample of monocytes is activated prior to, subsequent to or simultaneously upon contacting the same with MTX with an activating compound as defined in the first or second aspect of the invention. In another particular embodiment of the fourth aspect of the invention or any of its particular embodiments, cell metabolism is induced, prior to or simultaneously upon contacting the biological sample with MTX, by phytohemagglutinin.

In another particular embodiment of the fourth aspect of the invention or any of its particular embodiments, the biological sample is contacted with a concentration range of MTX identical to that identified in any of the first or second aspects of the invention.

A fifth aspect of the invention relates to a method for determining the response to methotrexate (MTX) of a subject diagnosed with RA from a monocyte sample isolated from said subject, wherein the subject is characterized in that the subject has not, prior to obtaining the sample, been treated with disease-modifying drugs (DMARDs), such as MTX, and wherein the method comprises:
a. Determining or measuring the cellular metabolic activity levels obtained *in vitro* from such biological sample isolated from activated monocytes, which have been or are in contact with a sufficient amount of methotrexate (MTX);
b. comparing said levels of step a) with a reference value indicative of the levels of MTX responders or healthy donors, where values, preferably significantly, higher than said reference value are indicative of non-response to treatment with MTX and that the subject is therefore a non-responder to said treatment. Otherwise, it will be understood that the subject is a responder to MTX.

In a particular embodiment of the fifth aspect of the invention, cellular metabolic activity is carried out with a fluorescence assay that measures cellular metabolic activity, such as with resazurin.

In another particular embodiment of the fifth aspect of the invention or any of its particular embodiments, the human subject from which the sample is obtained is characterized in that the subject has not been treated with MTX, and preferably in that the subject presents a DAS 28 ≥ 2.6.

In another particular embodiment of the fifth aspect of the invention or any of its particular embodiments, the biological sample comprises monocytes or a substantially pure population of monocytes.

In another particular embodiment of the fourth aspect of the invention or any of its particular embodiments, the biological sample of monocytes is activated prior to or simultaneously upon contacting the same with MTX with an activating compound as defined in any of the first or second aspects of the invention. In another particular embodiment of the fourth aspect of the invention or any of its particular embodiments, cell metabolism is induced, prior to or simultaneously upon contacting the biological sample with MTX, by phytohemagglutinin.

In another particular embodiment of the fourth aspect of the invention or any of its particular embodiments, the biological sample is contacted with a concentration range of MTX as identified in the first or second aspect of the invention.

A sixth aspect of the invention relates to a kit for implementing the method of the fifth aspect of the invention for determining the response to methotrexate (MTX) of a subject diagnosed with RA.

A seventh aspect of the invention relates to a method for screening, classifying or selecting patients as responders or non-responders to MTX, wherein said method replicates the steps of the method of the second or fifth aspect of the invention for determining response to MTX and classifies, screens or selects said sample and thus the patient or subject from which said response is derived as a responder or non-responder to MTX.

A seventh aspect of the invention relates to a composition comprising MTX, for use in the treatment of a patient diagnosed with rheumatoid arthritis and identified as a responder to such treatment according to the method of any one of the second, fifth or seventh aspects or according to any of their particular embodiments or

In the following, the following examples are merely illustrative of the present invention, but not limiting thereof.

### Example

Abbreviations used in the examples of the present invention.
RA: Rheumatoid arthritis.
MTX: Methotrexate.
PBMCs: Peripheral blood mononuclear cells.
PHA: phytohemagglutinin, T-cell mitogen .
PrestoBlue: resazurin vital probe.
HBSS: Isotonic salt buffer (Hank's Balanced Salt Solution).
ROS: Reactive Oxygen Species.

This example constitutes validation of two *in vitro* diagnostic assays/tests of response to treatment with methotrexate (MTX). The two assays are briefly described below:
1) ***"Monocyte" TEST:*** monocytes are key cells in the pathogenesis of diseases such as rheumatoid arthritis. Their activation and migration to the synovial membrane is a central phenomenon in the onset of inflammatory manifestations of the disease. They are cells of the innate immune system that have the capacity to bridge to adaptive immunity. These cells are probably one of the important targets for MTX. In this sense, the absolute number of monocytes in patients with rheumatoid arthritis who are non-responders should be increased (with respect to responders and healthy donors) at 6 months of treatment with MTX. However, the absolute number of monocytes may not be sensitive enough to detect interindividual differences with sufficient precision. Therefore, the decision was made to investigate a model of *in vitro* exposure of RA patients' cells to MTX and the measurement of its direct effect as a pharmacodynamic biomarker of response. This first test, therefore, consists of a monocyte culture that is exposed to MTX (except for an active control). The overall cellular metabolism of monocytes and their proliferation is then measured using resazurin, a known and validated redox-sensitive fluorochrome that changes color according to the "metabolic intensity" and the number of live cells present. Activated and proliferating immune system cells exhibit a much more intense metabolic state, making it a good marker of overall response. Since MTX should inhibit cell activation and proliferation, comparison between the overall metabolism of active control cells vs. those exposed to MTX should provide a measure of the effect of MTX on monocyte activation and proliferation, which would serve as a biomarker of clinical response to MTX in an example of RA.
2) ***ROS TEST:*** activated immune system cells typically generate reactive oxygen species, to a greater or lesser extent depending on cell type and function. This invention tests a casual and not necessarily predicted observation that increased redox activity is paradoxically shown in cultured PBMCs from MTX-responsive RA patient cells. Therefore, the hypothesis is that treatment of PBMCs with the drug methotrexate causes an increase in reactive oxygen species (ROS) production due to increased apoptosis in T lymphocytes in responder patients. This increase could serve as a biomarker of response to treatment in patients diagnosed with RA before starting said treatment.

Based on the above, in the present example the above two assays will be carried out using PBMCs obtained or isolated from RA patients. Briefly, first, a peripheral blood draw is performed from a rheumatoid arthritis patient who meets the inclusion criteria indicated below in the "Methodology" section, and peripheral blood mononuclear cells (PBMCs) are isolated by density gradient. These PBMCs are frozen and maintained in liquid nitrogen for a time period of at least two weeks. After this time, the PBMCs are thawed and incubated in a flat-bottomed p96 plate at 37°C and 5% CO₂ for 24 hours. The next day, phytohemagglutinin (PHA) and MTX are added to the corresponding wells and the plate is incubated for 48 hours. Finally, the probes corresponding to each test are added:
- ROS test: ROS probe + E.coli
- MONOCYTE test: resazurin

Both tests were carried out on the same plate, read at Ex/Em 535/610 wavelengths, in a fluorescence reader. The specific protocol followed in this case is detailed below as ROS + MONOCYTE protocol.

### - Methodology

### Study population

Naive patients who have not received treatment with MTX or any other DMARD and who present a DAS28 ≥ 2.6. Treatment with MTX is started at visit 1, after obtaining the blood sample used in the tests. There are three visits in the study that will coincide with routine clinic visits for these patients.
1. Visit 1: Drawing of 30 mL of blood in addition to those planned to be drawn from the patient for ongoing treatment. In addition to the main routine variables collected. Start of treatment with MTX.
2. Visit 2: Routine core variables collected.
3. Visit 3: Drawing of 30 mL of blood in addition to those planned to be drawn from the patient for ongoing treatment. In addition to the main routine variables collected.

### Inclusion criteria

- Age ≥ 18 years
- Men and women
- Rheumatoid arthritis diagnosed according to EULAR guideline criteria.
- Naive patients who have not received treatment with MTX or any other DMARD and who present a DAS 28 ≥ 2.6.

### Exclusion criteria

- Age < 18 years old
- Refusal to sign informed consent
- Active infection with systemic involvement by viruses, bacteria or fungi requiring antimicrobial treatment.
- Infection by HIV, hepatitis virus or other infectious agents that prevent proper handling of clinical samples in conventional laboratories.
- Reasonable diagnostic doubts that prevent the classification of the subject in any of the study groups.
- Patients who have not been able to receive, or have had to discontinue, treatment with a DMARD due to intolerance or contraindication.
- Patients who have been taking corticosteroids for ≥1 month and at a dose ≥ 15 mg prednisone or equivalent on the day before the date of study inclusion
- Patients with severe systemic diseases (other than RA) that may affect the patient's immune response, or physiological situations such as pregnancy or lactation that may also cause changes in said response.

### Main variables collected in the study

- Demographic data
- Complete blood count (obtained in routine analysis): total leukocytes and leukocyte differential count
- Basic blood biochemistry (obtained in routine analysis): creatinine, GOT/GPT, albumin
- Immunosuppressive drugs received by the patient (name of active ingredient, dosage in mg/day, duration of treatment)
- Dose of immunosuppressant in blood if available
- History of immunosuppressive drugs (name of active ingredient, dosage in mg/day, time on treatment with the drug, cause and date of change)
- RA Data:
   ∘ Date of diagnosis
   ∘ DAS28 on all visits (and date)
   ∘ Visceral involvement at the present time yes/no
   ∘ ESR, CRP and Rheumatoid Factor in last blood test (and date)
   ∘ Patient adherence to treatment questionnaire
   ∘ The recording of the SDAI measurement as the main variable to collect
   ∘ Boolean Remission Criteria (ACR/EULAR)
   ∘ Ultrasound /Echo-doppler to assess joint damage, following the standardized protocol for rheumatoid arthritis.

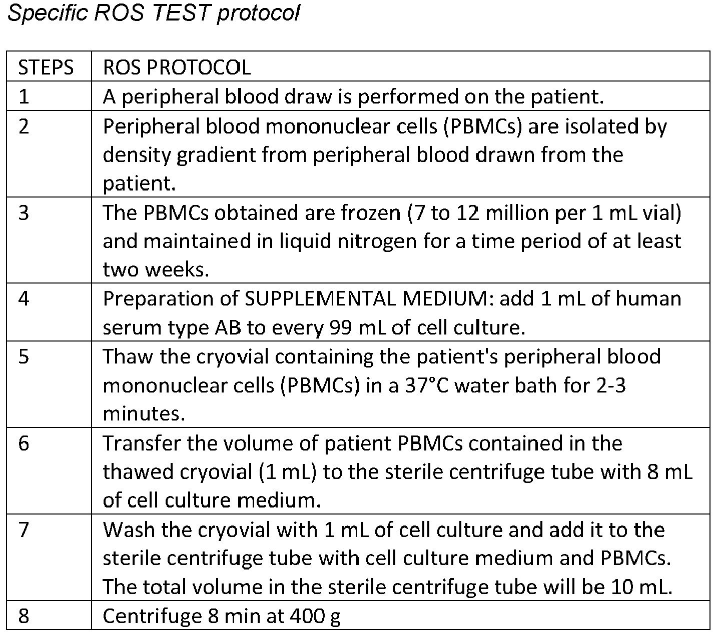

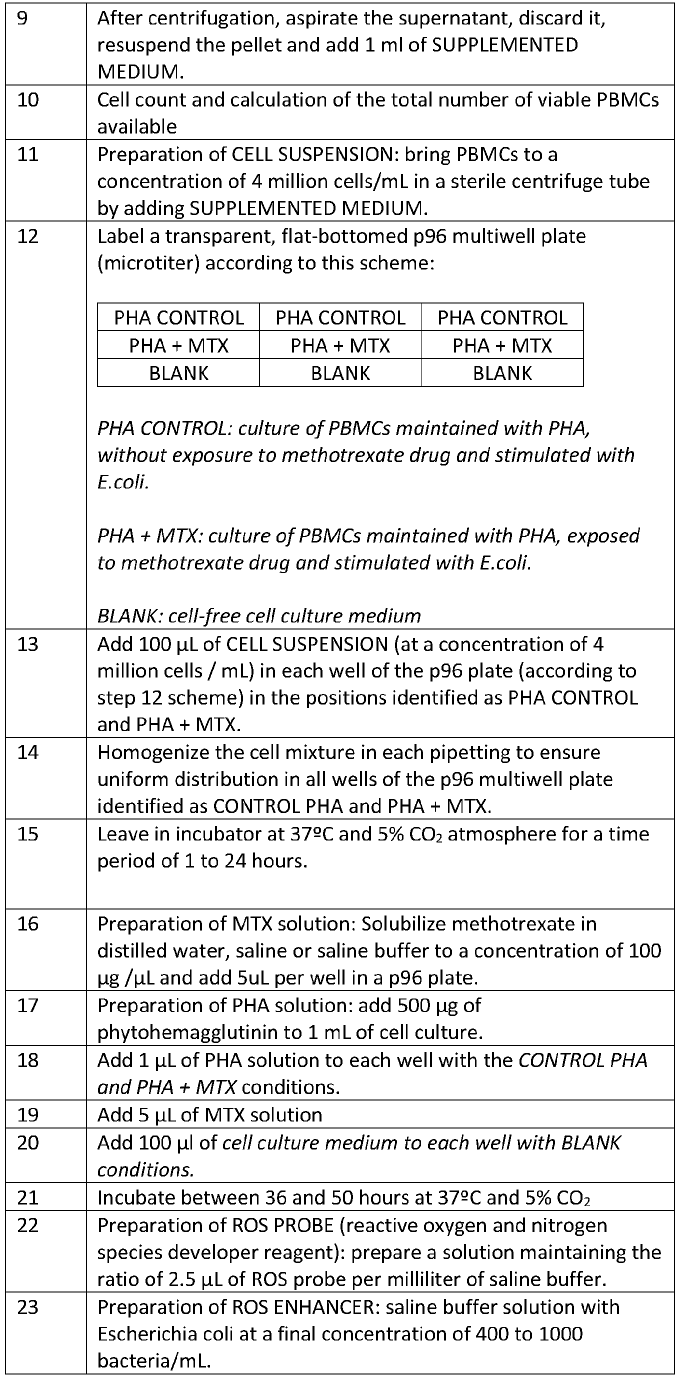

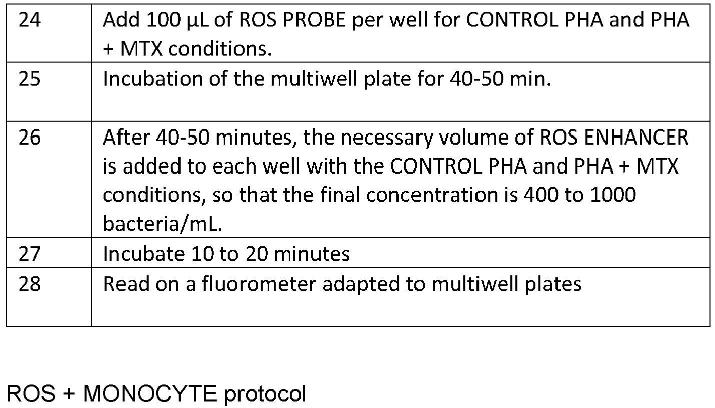

It is noted that this is the specific protocol that was carried out to generate the results shown throughout this patent specification.

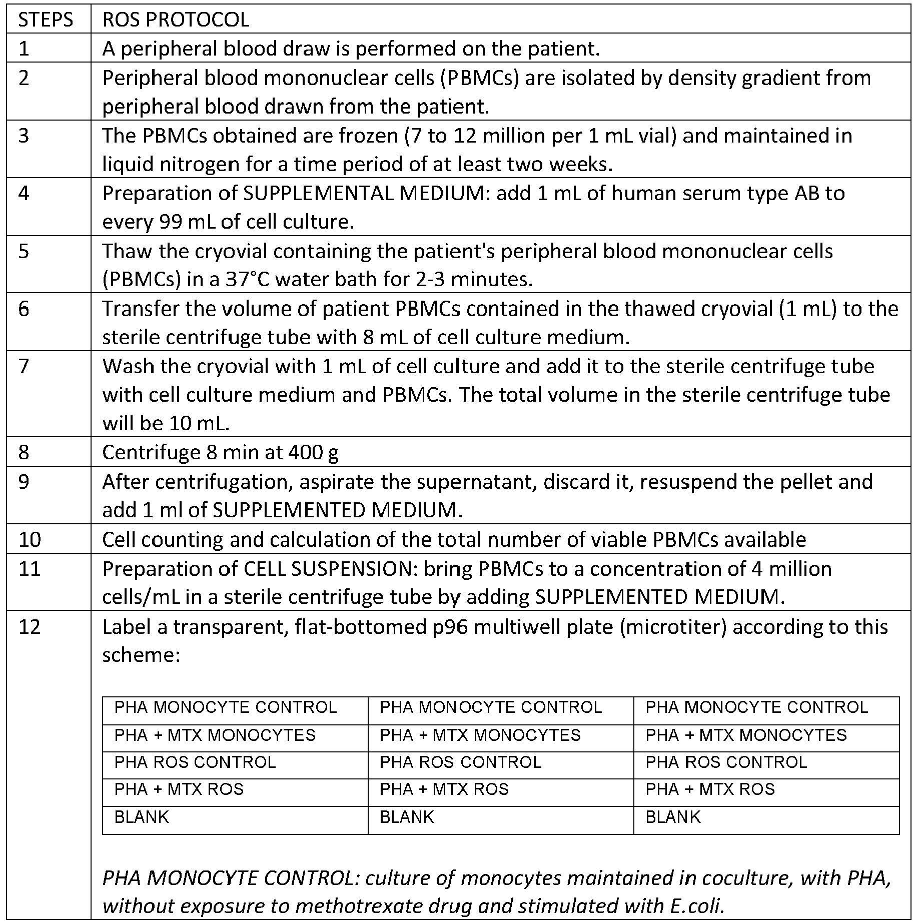

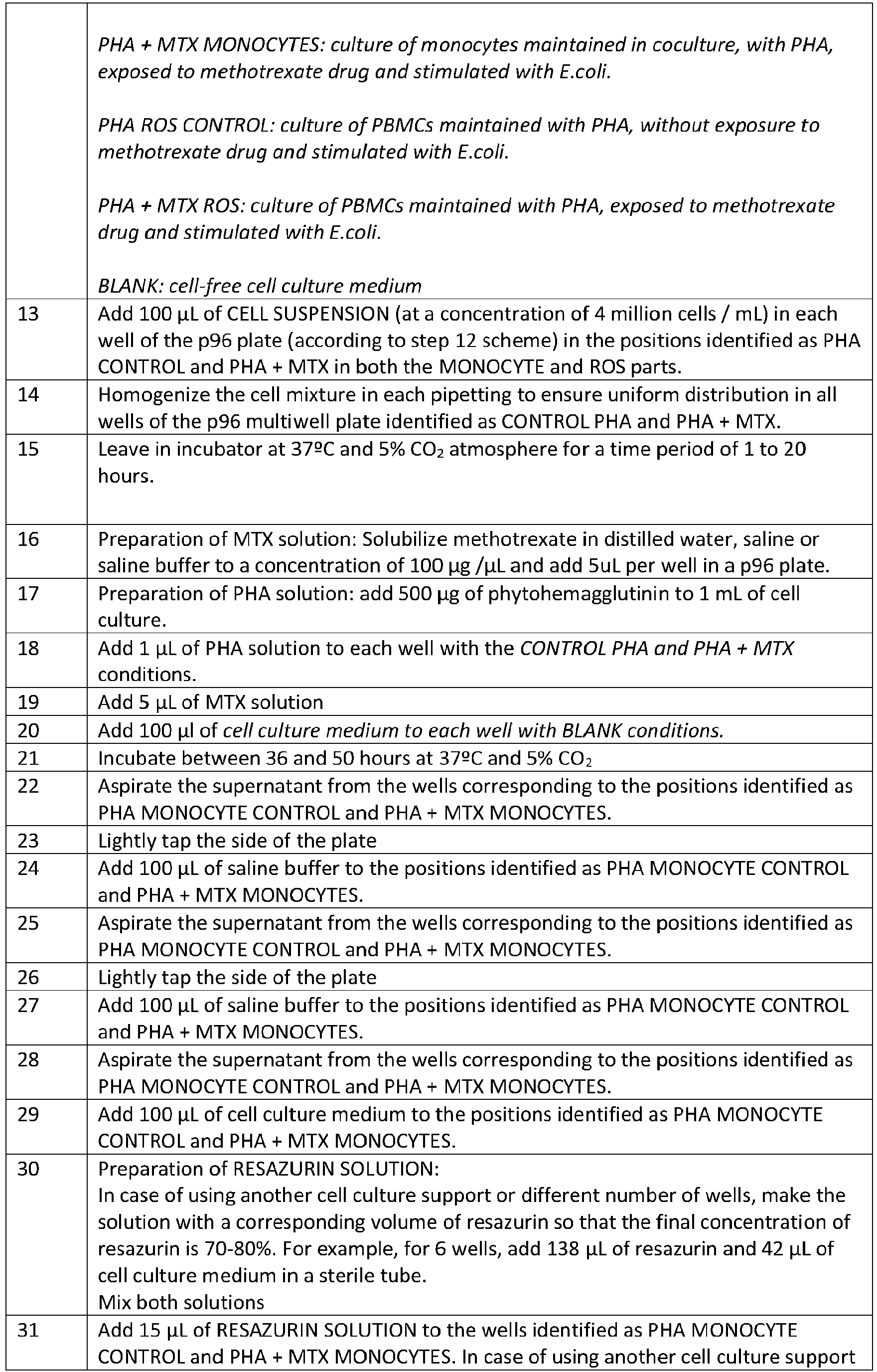

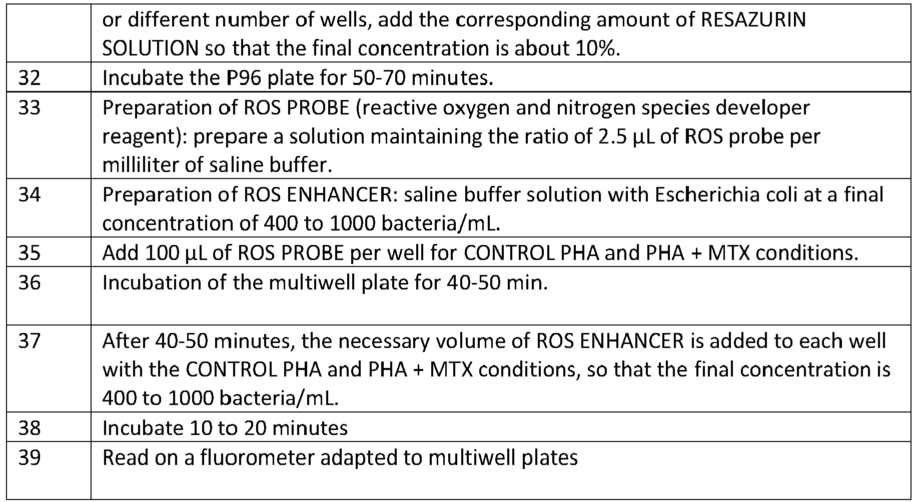

### - Results

### ROS Test

Figure 2 shows the data as plots for each group of patients showing the distribution of all patients. The normal distribution of the results was checked by the D'Agostino Pearson test. Comparisons and differences between groups were analyzed by ANOVA analysis. Specifically, ANOVA analysis showed a statistically significant difference between those naïve RA patients who did not present clinical remission at 6 months of treatment with methotrexate and those naïve RA patients who did present clinical remission at 6 months of treatment with methotrexate:

**ANOVA**

| Source | SS | DF | MS | F | p-value |
|---|---|---|---|---|---|
| OVERALL REM | 3.697280E+02 | 1 | 3.697280E+02 | 18.61 | 0.0003 |
| Error | 4.569808E+02 | | 19.868731568 | | |
| Total | 8.267088E+02 | | 34.446199589 | | |

All statistical analyses were performed with Analyse-it 5.92 software; p < 0.05 was considered statistically significant.

Figure 3 shows a ROC curve plot of the ROS test in patients with new-onset rheumatoid arthritis who did and did not present clinical remission in response to methotrexate. Each of the points represents a possible cut-off point of the diagnostic test and reports its respective sensitivity (y-axis) and specificity (x-axis). Both axes include values between 0 and 1. As can be seen from the data shown below, the AUC in Figure 3 presents a clearly discriminatory value greater than 0.90:

| | NO | YES | Total |
|---|---|---|---|
| Remission | | | |

| | AUC | 95% CI | SE |
|---|---|---|---|
| ROS | 0.919 | 0.813 to 1.025 | 0.0540 |

Therefore, from this data the following can be concluded:
- There is an association between ROS production in naïve patients and response to methotrexate.
- Patients able to metabolize MTX at low doses maintain the drug by-products intracellularly for a time, during which time ROS detectable by the test are generated.

### Monocyte Test

Figure 4 shows the percentage of monocytes from, NO: naive RA patients who do not present clinical remission at 6 months of treatment with methotrexate (n= 8), YES: naive RA patients who do present clinical remission at 6 months of treatment with methotrexate (n= 18). ANOVA analysis comparing these two groups revealed the following results:

**ANOVA**

| Source | SS | DF | MS | F | p-value |
|---|---|---|---|---|---|
| OVERALL REM | 1.748112E+03 | 1 | 1.748112E+03 | 7.14 | 0.0133 |
| Error | 5.877544E+03 | | 2.448976E+02 | | |
| Total | 7.625656E+03 | | 3.050262E+02 | | |

The p-value shown allows to conclude a clear discriminatory capacity of this test to differentiate between naive patients with RA who do not present clinical remission at 6 months of treatment with methotrexate and naive patients with RA who do present clinical remission at 6 months of treatment with methotrexate. This data is corroborated by Figure 5, which shows an AUC of 0.826 (discriminatory value).

| | NO | YES | Total |
|---|---|---|---|
| Remission | | | |

| | AUC | 95% CI | SE |
|---|---|---|---|
| %Monocytes | 0.826 | 0.664 to 0.989 | 0.0827 |

### References

1. Visser H, le Cessie S, Vos K, Breedveld FC, Hazes JM. How to diagnose rheumatoid arthritis early: a prediction model for persistent (erosive) arthritis. Arthritis Rheum. 2002 Feb;46(2):357-65. doi: 10.1002/art.10117. PMID: 11840437.

2. Saag KG, Teng GG, Patkar NM, et al. American College of Rheumatology 2008 recommendations for the use of nonbiologic and biologic disease-modifying antirheumatic drugs in rheumatoid arthritis. Arthritis Rheum. 2008 Jun 15;59(6):762-84. PMID: 18512708

3. Aletaha D, Ward MM, Machold KP, et al. Remission and active disease in rheumatoid arthritis: defining criteria for disease activity states. Arthritis Rheum. 2005 Sep;52(9):2625-36. PMID: 16142705

4. Mirshafiey A, Mohsenzadegan M. The role of reactive oxygen species in immunopathogenesis of rheumatoid arthritis. Iran J Allergy Asthma Immunol. 2008 Dec;7(4):195-202. PMID: 19052348.

5. Ji-Yeon Sung, Jang-Hee Hong, Hyung-Sik Kang, Inpyo Choi, Sang-Deok Lim, June-Kyu Lee, Jeong-Ho Seok, Jae-Heun Lee, Gang-Min Hur. Methotrexate suppresses the interleukin-6 induced generation of reactive oxygen species in the synoviocytes of rheumatoid arthritis. Immunopharmacology. Volume 47, Issue 1, 2000. Pages 35-44. ISSN 0162-3109

6. Elango T, Dayalan H, Gnanaraj P, Malligarjunan H, Subramanian S. Impact of methotrexate on oxidative stress and apoptosis markers in psoriatic patients. Clin Exp Med. 2014 Nov;14(4):431-7. doi: 10.1007/s10238-013-0252-7. Epub 2013 Aug 15. PMID: 23949337.

7. Amoruso A, Sola D, Rossi L, Obeng JA, Fresu LG, Sainaghi PP, Pirisi M, Brunelleschi S. Relation among anti-rheumatic drug therapy, CD14(+)CD16(+) blood monocytes and disease activity markers (DAS28 and US7 scores) in rheumatoid arthritis: A pilot study. Pharmacol Res. 2016 May;107:308-314. doi: 10.1016/j.phrs.2016.03.034. Epub 2016 Apr 1. PMID: 27045818.

8. Chara L, Sanchez-Atrio A, Perez A, et al. The number of circulating monocytes as biomarkers of the clinical response to methotrexate in untreated patients with rheumatoid arthritis. J Transl Med. 2015;13:2. Published 2015 Jan 16. doi:10.1186/s12967-014-0375-y. PMID: 25592233.

## Claims

1. Use of ROS (reactive oxygen species) production levels measured, preferably by fluorimetry or flow cytometry, *in vitro* from a biological sample that has been or is in contact with methotrexate (MTX), wherein the biological sample comprises peripheral blood mononuclear cells (PBMCs), or a population of T lymphocytes and/or monocytes, preferably a substantially pure population of activated T lymphocytes and/or monocytes, and isolated from a human subject diagnosed with rheumatoid arthritis and **characterized in that** the subject has not, prior to obtaining the sample, been treated with disease-modifying drugs (DMARDs), such as MTX, as a biomarker of response of said subject to methotrexate, wherein "response" of the subject to MTX is understood as the clinical remission of the subject at 6 months from the start of treatment with methotrexate.

2. The use according to the preceding claim, wherein the human subject from which the sample is obtained is **characterized in that** the subject has not been treated with MTX, and preferably **in that** the subject presents a DAS 28 ≥ 2.6.

3. The use according to any of the preceding claims, wherein the T lymphocytes and/or monocytes have been activated with T lymphocyte and/or monocyte activating compounds selected from the list consisting of PHA (phytohemagglutinin), bacterial antigens such as LPS (lipopolysaccharides), T lymphocyte stimulating antibodies (such as e.g. anti-CD3 and/or anti-CD28), Concanavalin A or phorbol esters (PMA) plus ionomycin

4. The use according to any of the preceding claims, wherein the biological sample is in contact or has been contacted with a concentration range of MTX in distilled water, saline or saline buffer of between 50 and 150 µg /µL, preferably of between 75 and 125 µg /µL, more preferably of between 90 and 110 µg /µL, still more preferably of about 100 µg /µL, for at least a time period of between 1 to 20 hours.

5. The use according to any of the preceding claims, wherein T lymphocytes and/or monocytes are activated prior to or simultaneously upon contact with MTX with phytohemagglutinin at a concentration of between 400-600 µg of phytohemagglutinin per mL of cell culture for at least a time period of between 1 to 20 hours.

6. The use according to any of the preceding claims, wherein ROS production is enhanced by LPS (lipopolysaccharide) and/or bacteria, such as E. coli, and ROS measurement is carried out via a fluorescence assay.

7. A method for determining the response to methotrexate (MTX) of a subject diagnosed with RA (rheumatoid arthritis) from a sample of PBMCs isolated from said subject, or a population of T lymphocytes and/or monocytes, preferably a substantially pure population of activated T lymphocytes and/or monocytes, wherein the subject is **characterized in that** the subject has not, prior to obtaining the sample, been treated with disease-modifying drugs (DMARDs), such as MTX, and wherein the method comprises:
a. Determining or measuring the ROS (reactive oxygen species) production levels of the biological sample, in contact with a sufficient amount of methotrexate (MTX), from peripheral blood mononuclear cells (PBMCs) or activated T lymphocytes and/or monocytes isolated from the human subject diagnosed with rheumatoid arthritis;
b. Comparing the result of the determination of step a) with a reference value indicative of ROS production in a subject or population of subjects not responding to MTX, where values, preferably significantly, higher than such reference value are indicative of response to treatment with MTX and that the subject is therefore a responder to such treatment.

8. The method according to the preceding claim, wherein the human subject from which the sample is obtained is **characterized in that** the subject has not been treated with MTX, and preferably **in that** the subject presents a DAS 28 ≥ 2.6.

9. The method according to any of claims 7 or 8, wherein the T lymphocytes and/or monocytes have been activated with T lymphocyte and/or monocyte activating compounds selected from the list consisting of PHA (phytohemagglutinin), bacterial antigens such as LPS (lipopolysaccharides), T lymphocyte stimulating antibodies (such as anti-CD3 and/or anti-CD28), Concanavalin A or phorbol esters (PMA) plus ionomycin.

10. The method according to any of claims 7 to 9, wherein the biological sample is in contact or has been contacted with a concentration range of MTX in distilled water, saline or saline buffer of between 50 and 150 µg /µL, preferably of between 75 and 125 µg /µL, more preferably of between 90 and 110 µg /µL, still more preferably of about 100 µg /µL, for at least a time period of between 1 to 20 hours.

11. The method according to any of claims 7 to 10, wherein T lymphocytes and/or monocytes are activated prior to or simultaneously upon contact with MTX with phytohemagglutinin at a concentration of between 400-600 µg of phytohemagglutinin per mL of cell culture for at least a time period of between 1 to 20 hours.

12. The method according to any one of claims 7 to 11, wherein ROS production is enhanced by LPS (lipopolysaccharide) and/or bacteria comprising LPS in their cell wall, such as E. coli, and ROS measurement is carried out via a fluorescence assay.

13. The method according to any of claims 7 to 12, wherein said method is for screening, classifying or selecting patients as responders or non-responders to MTX.

14. A composition comprising MTX, for use in the treatment of RA in a patient diagnosed with rheumatoid arthritis and identified as a responder according to the method of claim 13.

15. *In vitro* use of a kit suitable for implementing the method according to any of claims 7 to 12, for screening, classifying or selecting patients or patient samples as responders or non-responders to MTX.
